# FASCICULE DE BREVET EUROPEEN

(11) **EP 2 321 385 B1**
(45) Date de publication et mention de la délivrance du brevet: **31.05.2017**
(21) Numéro de dépôt: 09809384.2
(22) Date de dépôt: 29.07.2009
(51) Int. Cl.: C10G 63/04, C07C 2/12, C07C 4/06, C07C 11/06, C10G 11/18, C10G 69/04, C10G 50/00, C10G 11/05

(54) **PROCÉDÉ DE CONVERSION D'UNE CHARGE LOURDE EN ESSENCE ET EN PROPYLÈNE PRÉSENTANT UNE STRUCTURE DE RENDEMENT MODULABLE**
VERFAHREN ZUR UMWANDLUNG EINER SCHWEREN LADUNG IN BENZIN UND PROPYLEN MIT EINER STRUKTUR FÜR VARIABLEN ERTRAG
METHOD OF CONVERTING A HEAVY CHARGE INTO PETROL AND PROPYLENE, HAVING A VARIABLE-YIELD STRUCTURE

(30) Priorité: 29.08.2008 FR 0804759
(43) Date de publication de la demande: 18.05.2011
(73) Titulaire: IFP Énergies nouvelles, 92852 Rueil-Malmaison Cedex (FR)
(72) Inventeur: DIGNE, Romina, F-69007 Lyon (FR); CALLEBERT, Olivier, F-92500 Rueil Malmaison (FR); ROUX, Romain, F-92500 Rueil Malmaison (FR)
(86) Numéro de dépôt international: PCT/FR2009/000945
(87) Numéro de publication internationale: WO 2010/023369

(56) Documents cités:
- EP-A- 0 426 400
- WO-A-93/02025
- WO-A-03/078547
- US-A- 4 822 477

## Description

### DOMAINE DE L'INVENTION

L'invention concerne un procédé de conversion d'une charge hydrocarbonée lourde présentant une grande flexibilité sur la production d'essence et de propylène. Plus précisément, le procédé selon la présente invention permet de coproduire de l'essence avec un rendement minimum et du propylène avec un rendement pouvant atteindre 10 % poids de la charge.

Le rendement minimum en essence dépend de la charge de départ, mais sur des charges usuelles telles que des distillats sous vide ou des résidus atmosphériques, ce rendement minimum est supérieur à 40%, et préférentiellement supérieur à 45% poids, par rapport à la charge entrante.

La demande en propylène augmente fortement depuis de nombreuses années. La source principale de production de propylène est le vapocraquage de naphta. L'augmentation du rendement en propylène du procédé de vapocraquage étant limitée, les raffineurs sont incités à produire de plus en plus de propylène par le procédé de craquage catalytique connu sous le sigle FCC, abréviation de la terminologie anglo-saxonne "Fluid Catalytic Cracking", qui signifie Craquage Catalytique en lit Fluidisé.

Le procédé FCC permet de convertir des charges hydrocarbonées lourdes, dont la température d'ébullition initiale est généralement supérieure à 340°C en des fractions hydrocarbonées plus légères, notamment une coupe essence, par craquage des molécules de la charge lourde en présence d'un catalyseur acide.

Le FCC produit également des Gaz de Pétrole Liquéfiés (GPL) en quantité importante avec des teneurs en oléfines élevées.

La mise en oeuvre du craquage catalytique orientée vers la production de propylène repose soit sur des conditions opératoires plus sévères (augmentation de la température en sortie du réacteur FCC et circulation du catalyseur plus élevée), soit sur l'utilisation d'additifs spécifiques au catalyseur de craquage, soit sur une combinaison de ces deux moyens principaux.

Généralement le rendement en propylène est acquis au détriment du rendement en essence et est associé à des conditions opératoires relativement sévères.

Dans le procédé selon la présente invention, le rendement en essence est maintenu à une valeur minimum qui dépend bien entendu de la nature de la charge et du catalyseur utilisé, même lorsque les conditions opératoires sont déterminées en vue de maximiser la production de propylène.

Lorsque l'objectif de production de propylène disparaît, le procédé permet alors de maximiser la production d'essence.

Le procédé objet de la présente invention peut donc être globalement présenté comme un procédé de coproduction de propylène et d'essence avec un rendement minimum d'essence.

Le procédé met en oeuvre successivement des réactions de craquage catalytique, et d'oligomérisation des oléfines issues de la coupe C3/C4, ou de la coupe C4, ou de la coupeC4/C5), le terme Cn désignant une coupe d'hydrocarbures ayant n atomes de carbone. Ce procédé produit de l'essence avec un rendement minimum généralement supérieur à 40 % poids par rapport à la charge entrante, et du propylène avec un rendement modulable dans une large gamme, pouvant aller sur certaines charges jusqu'à 10 % poids.

La présente invention est compatible avec toutes les technologies de réacteur de craquage catalytique, que ce soit une technologie à écoulement gaz solide ascendant (appelé "riser" dans la terminologie anglo saxonne), ou une technologie à écoulement descendant (appelée "dropper" dans la terminologie anglo saxonne).

### EXAMEN DE L'ART ANTERIEUR

Le brevet FR 2 837 213 décrit un procédé de conversion d'une charge lourde qui comprend une étape de craquage catalytique, d'hydrogénation sélective et d'oligomérisation des oléfines à 4 et/ou 5 atomes de carbone issues du craquage catalytique. La charge lourde et les oligomères sont craquées ensemble ou séparément avec le même catalyseur. Les effluents de craquage sont séparés dans une zone de fractionnement commune. Une partie de la coupe C4 ou C4/C5 obtenue après fractionnement est oligomérisée. Lorsque la coupe C4 est oligomérisée puis craquée comme charge secondaire, ce procédé accroît le rendement en propylène tout en conservant le rendement en essence. Le procédé selon la présente invention permet comme le brevet cité d'augmenter le rendement en propylène tout en augmentant faiblement le rendement en essence, mais il offre de plus la possibilité d'augmenter très fortement le rendement en essence et la capacité de l'unité si la production de propylène n'est plus souhaitée et ceci avec la même installation et sans changer de catalyseur.

Le brevet FR 2 837 199 décrit un procédé de production de propylène et d'essence comprenant au moins une étape d'oligomérisation et une étape de craquage catalytique des oligomères formés. Les oligomères formés à partir d'oléfines à 4 et/ou 5 atomes de carbone sont craqués dans l'unité de craquage catalytique pour former du propylène en grande quantité. La charge du procédé selon le brevet cité est donc une charge hydrocarbonée "légère" de point d'ébullition inférieur à 340°C. Contrairement au procédé décrit dans le brevet FR 2 837 199, le procédé selon la présente invention concerne la conversion d'une charge lourde dont le point d'ébullition est supérieur à 340°C afin d'obtenir beaucoup d'essence et plus ou moins de propylène.

### DESCRIPTION SOMMAIRE DES FIGURES

La figure 1 représente un schéma du procédé selon l'invention dans la marche dite " maxi propylène".
La figure 2 représente un schéma du procédé selon l'invention dans la marche dite "maxi essence".

### DESCRIPTION SOMMAIRE DE L'INVENTION

L'invention concerne un procédé de conversion d'une charge hydrocarbonée dite lourde, c'est à dire constituée d'hydrocarbures de température d'ébullition supérieure à environ 340°C, en vue de coproduire du propylène et de l'essence avec un rendement minimum.

Le procédé selon l'invention comprend au moins deux étapes réactionnelles, une première étape de craquage catalytique et une seconde étape d'oligomérisation des oléfines en C3 et C4, ou des oléfines en C4, ou des oléfines en C4 et C5, issues du craquage catalytique.

Une troisième étape réactionnelle d'hydrogénation sélective d'oléfines peut être nécessaire dans certains cas avant l'oligomérisation.

Le procédé selon l'invention permet de réaliser deux types de production correspondant à deux cas de marche distincts:
▪ Une marche dite "maxi propylène" correspondant à une production maximum en propylène tout en maintenant un rendement minimum en essence, voire même légèrement augmenté par rapport au rendement potentiel de l'unité de craquage catalytique seule ou,
▪ Une marche dite "maxi essence" correspondant à une production maximale en essence sans production de propylène.

Un des intérêts de l'invention est de pouvoir basculer au cours du temps de l'une à l'autre des deux marches précédemment définies. Il est également possible d'opérer l'unité dans n'importe quel mode intermédiaire entre les marches "maxi propylène" et "maxi essence".

Ce basculement est très simple à opérer puisqu'il consiste essentiellement à modifier la charge entrante à l'unité d'oligomérisation, sans modification substantielle des conditions opératoires de l'unité de craquage catalytique et de l'unité d'oligomérisation, et bien entendu, sans modification des catalyseurs utilisés.

### En mode "maxi propylène":

▪ La charge lourde est craquée dans un réacteur de craquage catalytique en lit fluidisé en présence d'un catalyseur de craquage.
▪ Une deuxième charge relativement légère est craquée avec le même catalyseur de craquage, séparément ou en mélange avec la charge lourde. Cette deuxième charge comprend des oléfines à au moins 8 atomes de carbone ayant été produites par oligomérisation des oléfines à 4 (ou à 4 et 5) atomes de carbone issues du craquage catalytique. On parle dans la suite de coupe C4 oléfinique ou de coupe C4/C5 oléfinique.
▪ Les effluents du craquage des deux charges sont envoyés dans une zone de fractionnement commune, et le catalyseur utilisé pour le craquage des deux charges est régénéré dans une zone de régénération commune.
▪ La coupe C4 (ou C4/C5) oléfinique issue du craquage catalytique est envoyée dans une unité d'oligomérisation. Une hydrogénation sélective de la coupe C4 (ou C4/C5) avant l'unité d'oligomérisation peut être nécessaire ainsi qu'un traitement préalable pour retirer les impuretés telles que les composés azotés et soufrés. Les oligomères formés sont en partie envoyés au craquage catalytique pour produire du propylène car ce sont de bons précurseurs de propylène. Le reste des oligomères non recyclés est mélangé à la coupe essence produite par le craquage catalytique.

### En mode "maxi essence":

▪ La charge lourde est craquée dans le réacteur de craquage catalytique en présence du même catalyseur de craquage qu'en mode maxi propylène. Comme il n'y a pas d'autre charge craquée, une quantité de charge lourde supérieure à celle du mode maxi propylène peut être convertie.
▪ La coupe C3/C4 issue du craquage catalytique est envoyée dans une unité d'oligomérisation. Une hydrogénation sélective de la coupe C3/C4 avant l'unité d'oligomérisation peut être nécessaire.
▪ Les oligomères formés en C6+ sont séparés en une coupe essence qui est mélangée à la coupe essence produite par le craquage catalytique, et une coupe plus lourde qui peut être incorporée au pool gazole.

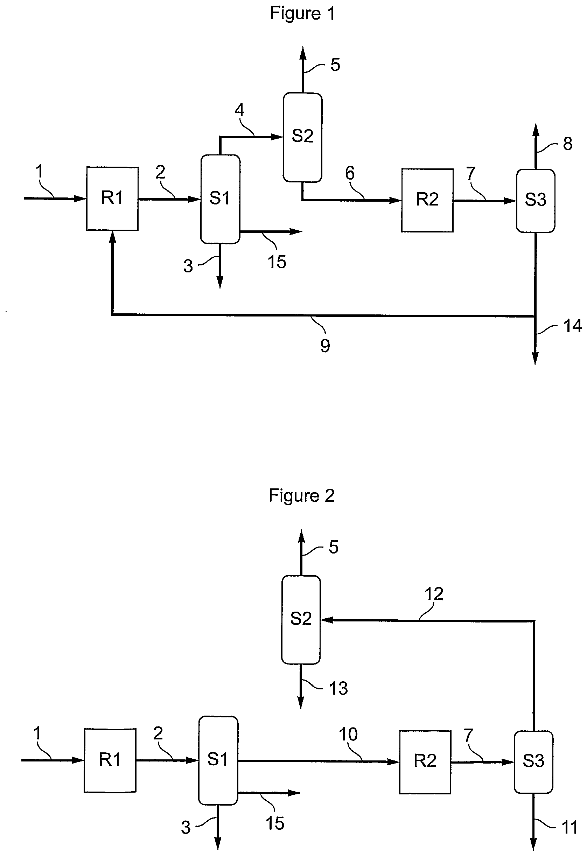

Comme on peut le constater sur les schémas du procédé (figure 1 pour la marche maxi propylène, figure 2 pour la marche maxi essence), le basculement d'une marche à l'autre est très simple. Par exemple, pour passer de la marche "maxi propylène" à la marche "maxi essence", il suffit d'envoyer à l'unité d'oligomérisation la coupe oléfinique C3 + C4 (et non plus seulement la coupe C4 ou la coupe C4+C5), et ne plus recycler au craquage catalytique les oligomères formés.

De manière plus précise, le procédé de coproduction d'essence et de propylène à partir d'une charge hydrocarbonée de point d'ébullition initial supérieur à 340°C selon l'invention fait appel à une unité de craquage catalytique suivie d'une unité d'oligomérisation pouvant fonctionner selon deux marches dite "maxi propylène" (marche 1), et "maxi essence" (marche 2) dans lequel,
- pour la marche "maxi propylène", la charge de l'unité d'oligomérisation est constituée de la coupe C4 ou de la coupe C4/C5 oléfinique issue du craquage catalytique, et les effluents de l'unité d'oligomérisation sont séparés en une coupe d'oligomères en C8+ qui est au moins en partie recyclée à l'entrée de l'unité de craquage catalytique, la coupe essence du procédé étant constituée de la coupe essence produite à l'unité de craquage catalytique et de la coupe oligomères issue de l'unité d'oligomérisation qui n'est pas recyclée au craquage catalytique,
- pour la marche "maxi essence", la charge de l'unité d'oligomérisation est constituée de la coupe C3/C4 oléfinique issue du craquage catalytique, et les effluents de l'unité d'oligomérisation sont séparés en une coupe d'oligomères en C6+ qui est ajoutée à la coupe essence issue du craquage catalytique pour constituer l'essence produite par le procédé, le propylène étant obtenu dans les deux marches à partir des effluents du FCC après séparation dans une ou plusieurs colonnes à distiller.

Comme exposé dans le paragraphe suivant, l'unité de craquage catalytique peut se décliner selon plusieurs modalités avec un seul réacteur traitant la charge lourde et la charge légère ou deux réacteurs traitant l'un la charge lourde et l'autre la charge légère. De plus chaque réacteur peut fonctionner en écoulement ascendant ou en écoulement descendant.

### DESCRIPTION DETAILLEE DE L'INVENTION

Selon l'invention, la charge globale à craquer contient plus de 50 % poids d'hydrocarbures ayant un point d'ébullition supérieur à 340°C. Généralement la charge est constituée d'un distillat sous vide, ou éventuellement d'un résidu atmosphérique. La charge globale craquée peut contenir jusqu'à 100 % poids d'hydrocarbures ayant un point d'ébullition supérieur à 340°C pour le mode "maxi essence". Pour le mode "maxi propylène" la charge contient souvent plus de 60 % poids, et le plus souvent plus de 70 %, par exemple entre 70 % et 95 % poids, d'hydrocarbures ayant un point d'ébullition supérieur à 340°C.

La charge secondaire du craquage catalytique nécessaire dans le mode "maxi propylène" contient généralement de 2 % à 40 % poids, le plus souvent de 4% à 30% poids, et de manière préférée de 6% à 25 % poids d'une coupe riche en oléfines constituée essentiellement d'oléfines à 8 atomes de carbone ayant été produites par oligomérisation des oléfines à 4 (ou 4 et 5) atomes de carbone. Cette coupe d'oligomères peut également contenir des quantités non négligeables de paraffines.

La charge secondaire peut aussi comprendre d'autres oligomères formés essentiellement à partir d'oléfines en C2 à C10.

Selon l'invention, le catalyseur de craquage est le même pour le mode "maxi propylène" et "maxi essence". Il s'agit d'un catalyseur constitué d'une zéolithe de type Y ultra stable dispersée dans une matrice d'alumine, de silice, ou de silice alumine, à laquelle on ajoute un additif à base de zéolithe ZSM5, la quantité en cristaux de ZSM5 dans l'inventaire total de l'unité de craquage étant inférieure à 10% poids.

L'invention peut donc se définir comme un procédé de coproduction d'essence et de propylène à partir d'une charge hydrocarbonée lourde de point d'ébullition initial supérieur ou égal à 340°C, ledit procédé faisant appel à une unité de craquage catalytique suivie d'une unité d'oligomérisation pouvant fonctionner selon deux marches dite "maxi propylène" (marche 1), et "maxi essence" (marche 2), procédé dans lequel,
- pour la marche "maxi propylène", la charge de l'unité d'oligomérisation est constituée de la coupe C4 ou de la coupe C4/C5 oléfinique issue du craquage catalytique, et les effluents de l'unité d'oligomérisation sont séparés en une coupe d'oligomères en C8+ qui est au moins en partie recyclée à l'entrée de l'unité de craquage catalytique, la coupe essence du procédé étant constituée de la coupe essence produite à l'unité de craquage catalytique et de la coupe oligomères issue de l'unité d'oligomérisation qui n'est pas recyclée au craquage catalytique,
- pour la marche "maxi essence", la charge de l'unité d'oligomérisation est constituée de la coupe C3/C4 oléfinique issue du craquage catalytique, et les effluents de l'unité d'oligomérisation sont séparés en une coupe d'oligomères en C6+ qui est ajoutée à la coupe essence issue du craquage catalytique pour constituer l'essence produite par le procédé,
le propylène étant obtenu dans les deux marches à partir des effluents du FCC après séparation dans une ou plusieurs colonnes à distiller.

L'unité de craquage catalytique peut comporter un seul réacteur traitant à la fois la charge lourde et la charge légère, ou deux réacteurs distincts traitant l'un la charge lourde, l'autre la charge légère. De plus, chacun des réacteurs peut être à écoulement ascendant ou à écoulement descendant. Le plus souvent, les deux réacteurs auront le même mode d'écoulement.

### A) Dans la marche "maxi propylène",

1) Lorsque le craquage catalytique est effectué dans un seul réacteur à écoulement ascendant, la température de sortie réacteur (ROT) est comprise entre 510 et 580°C, préférentiellement comprise entre 540 et 590°C, et le rapport C/O est compris entre 8 et 20.
2) Lorsque le réacteur est à écoulement descendant, la température de sortie du réacteur (ROT) est comprise entre 550°C et 700°C et le rapport C/O est compris entre 15 et 50.
3) lorsque le craquage catalytique est effectué dans deux réacteurs FCC distincts à écoulement ascendant, le premier réacteur FCC effectuant le craquage de la charge lourde travaille à une température de sortie réacteur (ROT1) comprise entre 510°C et 580°C, de préférence comprise entre 530 et 560°C, et un rapport C/O compris entre 5 et 10, et le second réacteur FCC effectuant le craquage des oligomères en C8+ issus de l'unité d'oligomérisation (dite charge légère), travaille à une température de sortie réacteur (ROT2) comprise entre 550°C et 650°C, préférentiellement comprise entre 570 et 620°C, avec un rapport C/O compris entre 8 et 25.
4) Lorsque le craquage catalytique est effectué dans deux réacteurs FCC distincts à écoulement descendant, le premier réacteur FCC effectuant le craquage de la charge lourde travaille à une température de sortie réacteur (ROT1) comprise entre 550°C et 700°C avec un rapport C/O compris entre 15 et 50, et le second réacteur FCC effectuent le craquage des oligomères en C8+ issus de l'unité d'oligomérisation (dite charge légère), travaille à une température de sortie réacteur (ROT2) comprise entre 570°C et 700°C, avec un rapport C/O compris entre 15 et 50.

### B) Dans la marche "maxi essence",

1) Lorsque le craquage catalytique est effectué dans un ou deux réacteurs travaillant en écoulement ascendant, la température de sortie (ROT) de chaque réacteur de craquage est comprise entre 500°C et 580°C, préférentiellement comprise entre 520°C et 550°C, et le rapport C/O est compris entre 5 et 10.
2) Lorsque le craquage catalytique est effectué dans un ou deux réacteurs travaillant en écoulement descendant, la température de sortie (ROT) de chaque réacteur de craquage est comprise entre 530°C et 650°C, et le rapport C/O est compris entre 15 et 25.

Les flux de catalyseur usé issus des deux réacteurs FCC sont séparés des effluents de craquage par tout système de séparation gaz solide connu de l'homme du métier et régénérés dans une zone de régénération commune.

L'effluent du réacteur de craquage catalytique, (ou bien les deux effluents s'il y a deux réacteurs FCC), est envoyé dans une zone de fractionnement pour produire plusieurs coupes dont une coupe essence et une coupe contenant des oléfines :
▪ à 4 (ou 4 et 5) atomes de carbone pour le mode "maxi propylène",
▪ à 3 et 4 atomes de carbone pour le mode "maxi essence".

Cette coupe à 3 et 4 (notée C3/C4), ou 4 (notée C4), ou 4 et 5 (notée C4/C5) atomes de carbone est envoyée à l'oligomérisation. Il est généralement préférable de soumettre cette coupe à une hydrogénation sélective pour réduire les composés dioléfiniques et/ou acétyléniques éventuellement présents afin d'augmenter la durée de cycle de l'oligomérisation. L'unité de séparation constituée d'une ou plusieurs colonnes à distiller est ajustée pour permettre l'extraction de la coupe C4 ou C4 /C5) ou de la coupe C3/C4.

En mode "maxi essence", la majeure partie ou la totalité des oligomères produits est ajoutée à la coupe essence obtenue par fractionnement de l'effluent du craquage catalytique. Le rendement en essence, rapporté à la quantité d'hydrocarbures ayant un point d'ébullition supérieur à 340°C est alors généralement compris entre 35 % et 65 % poids, souvent compris entre 50 % et 60 % poids. Le rendement en propylène, rapporté à la quantité d'hydrocarbures ayant un point d'ébullition supérieur à 340°C, est alors généralement inférieur à 1 % poids et ce propylène n'est en général pas récupéré spécifiquement.

En mode "maxi propylène", une partie, c'est à dire au moins 30 %, et préférentiellement la totalité des oligomères produits est recyclée au craquage catalytique. Le rendement en essence, rapporté à la quantité d'hydrocarbures ayant un point d'ébullition supérieur à 340°C est alors généralement compris entre 35 % et 55 % poids, le plus souvent compris entre 40 % et 50 % poids. Le rendement en propylène, rapporté à la quantité d'hydrocarbures ayant un point d'ébullition supérieur à 340°C, est alors généralement compris entre 4 % et 20 % poids, souvent compris entre 5 % et 15 %, et le plus souvent compris entre 7 % et 12 % poids.
Les conditions particulières des différentes étapes du procédé selon l'invention sont décrites ci-après plus en détail.

### 1) Craquage catalytique (FCC) :

Le catalyseur du réacteur FCC est typiquement mis en oeuvre sous forme de poudre fine de diamètre moyen des particules est généralement compris entre 40 et 140 micromètres, et le plus souvent compris entre 50 et 120 micromètres.

Le catalyseur de craquage catalytique contient au moins une zéolithe de type Y dispersée dans une matrice appropriée telle que l'alumine, la silice ou la silice-alumine.

Le catalyseur peut comprendre en outre au moins une zéolithe présentant une sélectivité de forme de l'un des types structuraux suivants : MEL (par exemple ZSM-11), MFI (par exemple ZSM-5), NES, EUO, FER, CHA (par exemple SAPO-34), MFS, MWW, ou peut également être l'une des zéolithes suivantes: NU-85, NU-86, NU-88 et IM-5, qui présentent également une sélectivité de forme. L'avantage de ces zéolithes présentant une sélectivité de forme est l'obtention d'une meilleure sélectivité propylène / isobutène, c'est à dire un rapport propylène / isobutène plus élevé dans les effluents de craquage.

La proportion de zéolithe présentant une sélectivité de forme par rapport à la quantité totale de zéolithe peut varier en fonction des charges utilisées et de la structure des produits recherchés. Souvent, on utilise de 2 à 60 %, préférentiellement de 3 à 40 %, et en particulier de 3 à 30 % poids de zéolithe (s) présentant une sélectivité de forme.

La ou les zéolithes peuvent être dispersées dans une matrice à base de silice, d'alumine ou de silice alumine, la proportion de zéolithe (toutes zéolithes confondues) par rapport au poids du catalyseur étant souvent comprise entre 3% et 80% poids, de préférence entre 4% et 50% poids et par exemple entre 5% et 25 % poids. Dans le cas ou plusieurs zéolithes sont utilisées, elles peuvent être incorporées dans une seule matrice ou dans plusieurs matrices différentes. La teneur en zéolithe présentant une sélectivité de forme dans l'inventaire totale est inférieure à 10% poids.

Le catalyseur utilisé dans le réacteur de craquage catalytique peut être constitué d'une zéolithe de type Y ultra stable dispersée dans une matrice d'alumine, de silice, ou de silice alumine, à laquelle on ajoute un additif à base de zéolithe ZSM5, la quantité en cristaux de ZSM5 dans l'inventaire total étant inférieure à 10% poids.

L'unité de séparation des effluents du réacteur de craquage catalytique (FCC) comporte généralement une séparation primaire des effluents du FCC, une section de compression et de fractionnement des gaz ainsi que des distillations pour le fractionnement des différentes coupes liquides.

Ce type d'unité de fractionnement est bien connu de l'homme du métier. Selon l'objectif visé En "maxi propylène", on sépare la coupe C4, ou la coupeC4/C5, des effluents pour l'envoyer à l'oligomérisation, et si besoin à l'hydrogénation sélective avant l'oligomérisation.

En marche maxi "essence", on sépare la coupe C3/C4 des effluents pour l'envoyer à l'oligomérisation, et si besoin à l'hydrogénation sélective avant l'oligomérisation.

Les coupes oléfiniques issues du craquage catalytique qui sont envoyées à l'oligomérisation peuvent être mélangées à des coupes riches en oléfines importées d'autres unités comme par exemple des coupes C3/C4 ou C4 provenant d'unité de cokéfaction, de vapocraquage, de conversion de méthanol en oléfines, etc.

Pour la coupe C4, on peut extraire l'isobutène, par exemple par éthérification de l'isobutène par un alcool, puis par distillation, pour éviter ou limiter la présence d' isobutène dans l'étape d'oligomérisation, ce composé tendant à former des isomères pouvant recraquer en isobutène au niveau du FCC, ce qui tend à produire une accumulation de ce composé si l'on ne réalise pas une purge suffisante d'isobutène.

On peut également, après extraction de l'isobutène, réaliser une distillation extractive, par exemple avec un solvant qui peut être La N-méthyl pyrrolidone (NMP) ou le Di-méthyl sulfoxyde (DMSO) ou un isomère de ce dernier, de manière à extraire la fraction insaturée des paraffines de la charge qui se retrouvent en mélange avec le solvant. Ceci permet de pouvoir recycler à l'hydrogénation sélective ou à l'oligomérisation un mélange de butènes (voire butènes/pentènes) ou propylène/butènes débarrassé des paraffines en C4 (voire C4/C5) ou C3/C4.

### 2) Hydrogénation sélective (facultative)

La coupe C4 ou la coupe C4/C5 ou la coupe C3/C4 des effluents du FCC contient en quantité faible des diènes (dioléfines) et des acétyléniques qui augmentent le cokage du catalyseur d'oligomérisation et donc diminuent la durée de cycle du réacteur d'oligomérisation. L'hydrogénation sélective des diènes et acétyléniques en mono-oléfines est donc préférable dans le procédé selon l'invention, sans être néanmoins indispensable.

L'objet principal de cette étape est de transformer les dioléfines (ou diènes) en mono-oléfines. En effet, les mono-oléfines sont la source des oligomères produits dans l'étape 3 d'oligomérisation. Un autre objet de cette étape est d'éliminer les traces d'hydrocarbures acétyléniques présentes dans ces coupes qui sont des composés indésirables pour l'oligomérisation. Les composés acétyléniques sont également transformés en mono-oléfines. La teneur résiduelle en dioléfines + acétyléniques de l'effluent de l'hydrogénation sélective est typiquement inférieure à environ 1000 ppm poids, de préférence inférieure à 100 ppm poids, et de façon très préférée inférieure à 20 ppm poids.

Généralement, cette étape d'hydrogénation sélective est réalisée en utilisant un catalyseur comprenant au moins un métal choisi dans le groupe formé par le nickel, le palladium, et le platine, déposé sur un support comprenant de l'alumine, de la silice ou de la silice-alumine. La teneur en palladium sur le support peut être typiquement de 0,01 % à 5% poids, de préférence de 0,05% à 1% poids.

La température opératoire de l'hydrogénation sélective est généralement comprise entre 0 et 200°C, la pression typiquement comprise entre 0,1 et 5 MPa, souvent entre 0,5 et 5 MPa, la vitesse spatiale typiquement comprise entre 0,5 et 20 m3 par heure et par m3 de catalyseur, préférentiellement entre 0,5 et 5 m3 par heure et par m3 de catalyseur, et le rapport molaire H2/(composés acétyléniques + dioléfiniques) généralement compris entre 0,5 et 5 et de préférence entre 1 et 3.

Pour réaliser l'hydrogénation sélective on fait appel à un ou plusieurs réacteurs en lit fixe, traversé à co-courant descendant par la charge à traiter et de l'hydrogène (ou un gaz contenant une fraction molaire notable d'hydrogène, par exemple d'au moins 50%), ou à courant descendant pour la charge à traiter et à courant ascendant pour l'hydrogène (ou le gaz riche en hydrogène).

Lorsque la coupe C5 est envoyée à l'oligomérisation, il est possible d'hydrogéner sélectivement cette coupe dans une unité séparée de l'unité d'hydrogénation des coupes C3 et C4, comme par exemple dans une unité d'hydrogénation sélective d'essence.

### 3) Oligomérisation

L'objet de cette étape est d'oligomériser les oléfines en C4 ou en C4/C5 (marche "maxi propylène") ou les oléfines en C3/C4 (marche "maxi essence"), pour obtenir un mélange d'hydrocarbures contenant des mono-oléfines avec un nombre d'atomes de carbone majoritairement supérieur ou égal à huit. Typiquement, à partir d'oléfines C4, on obtient des oligomères dont le nombre d'atomes de carbone est en grande partie inférieur ou égal à 30, et pour la plus grande partie compris entre 8 et 20.

L'oligomérisation se distingue de la polymérisation par une addition de molécules en nombre limité, le nombre de molécules s'additionnant étant dans le contexte de l'invention compris entre 2 et 10, bornes comprises, et généralement entre 2 et 5, notamment entre 2 et 4.

Les oligomères peuvent cependant comprendre des traces d'oléfines ayant été oligomérisées avec un nombre de molécules supérieur à 10. Le plus souvent, ces traces représentent moins de 5 % poids par rapport aux oligomères formés.

L'oligomérisation peut être réalisée en une ou plusieurs étapes, avec un ou plusieurs réacteurs et un ou plusieurs catalyseurs. La description suivante du catalyseur et des conditions opératoires peut s'appliquer à l'une quelconque des étapes et/ou à l'un quelconque des réacteurs.
- En marche maxi propylène, le catalyseur d'oligomérisation utilisé est préférentiellement un catalyseur à base de silice alumine. La température est comprise entre 100°C et 200°C, et préférentiellement comprise entre 140°C et 160°C. La pression est comprise entre 0,5 et 10 MPa.
- En marche maxi essence, le catalyseur d'oligomérisation utilisé est préférentiellement un catalyseur à base de silice alumine. La température opératoire est comprise entre 100°C et 200°C, et préférentiellement comprise entre 140°C et 160°C. La pression opératoire est comprise entre 2 et 10 MPa (1 MPa = 10⁶ Pascal).

L'invention est explicitée plus en détail au moyen de la description des figures 1 et 2.

La figure 1 représente l'installation pour la mise en oeuvre du procédé selon l'invention en marche opératoire "maxi propylène".

La charge de distillat sous vide ou de résidu atmosphérique est introduite par la ligne 1.

Le réacteur de craquage catalytique (R1) est alimenté par deux lignes séparées 1 et 9.

Les effluents du réacteur de craquage catalytique (R1) sont évacués par la ligne 2 et sont introduits dans une zone de séparation S1.

La zone de séparation S1 comprend typiquement un compresseur de gaz et des moyens de distillation/absorption.

La coupe C3+C4 des effluents du réacteur de craquage catalytique (R1) est envoyée vers la zone de séparation S2 par la ligne 4. L'essence correspondant à une coupe C5-220°C est évacuée par la ligne 15, et les autres effluents du FCC sont évacués par la ligne 3.

La zone de séparation S2 comprend typiquement des moyens de distillation. La coupe C3 contenant majoritairement du propylène est évacuée par la ligne 5. La coupe C4 très oléfinique est envoyée dans le réacteur d'oligomérisation R2 par la ligne 6.

Les effluents de l'oligomérisation sont extraits par la ligne 7 et introduits dans une zone de séparation S3. La zone de séparation S3 comprend typiquement une distillation des effluents d'oligomérisation pour récupérer les oligomères plus lourds, et la coupe C4 n'ayant pas réagi. La coupe C4 est constituée majoritairement de composés paraffiniques et d'une minorité de composés oléfiniques non transformés. Cette coupe C4 est évacuée par la ligne 8.

Les oligomères en C8 + sont en partie, et préférentiellement en totalité, introduits dans le réacteur de craquage catalytique R1 par la ligne 9, la ligne 14 correspondant aux oligomères non recyclés dans R1. Dans le cas où les oligomères ne sont pas envoyés en totalité au craquage catalytique, la majeure partie des oligomères restants sont mélangés à l'essence issue du craquage catalytique. Le craquage des oligomères dans R1 permet d'augmenter le rendement en propylène de l'installation.

La figure 2 représente l'installation pour la mise en oeuvre du procédé selon l'invention en marche opératoire "maxi essence".

Le réacteur de craquage catalytique R1 est alimenté par du distillat sous vide ou du résidu atmosphérique par la ligne 1.

La coupe C3+C4 des effluents du FCC est envoyée vers le réacteur d'oligomérisation R2 par la ligne 10.

La coupe C3+C4 n'ayant pas réagi dans R2 est envoyée vers la zone de séparation S2 par la ligne 12. La coupe C4 séparée de la coupe C3 dans S2 est évacuée par la ligne 13.

Les oligomères formés en C6+ ne sont pas recyclés et forment une coupe essence (ligne 11) qui rejoint la coupe essence issue du réacteur de craquage catalytique (ligne 15). L'essence produite par le procédé est donc constituée de la réunion des effluents des lignes 15 et 11.

### EXEMPLES

On fournit ci dessous 3 exemples pour illustrer la flexibilité améliorée du procédé par rapport aux procédés selon l'art antérieur.

### Exemple 1 (selon l'art antérieur)

On traite dans une unité de craquage catalytique de type FCC fonctionnant selon deux modes de marche (maxi essence et maxi propylène), une charge qui est un résidu atmosphérique dont les caractéristiques principales sont les suivantes :

| | |
|---|---|
| Densité | 0,93 |
| Viscosité à 50°C (cSt) | 84 |
| Carbone Conradson | 4,36 |
| Hydrogène (% poids) | 12,3 |
| TBP 10% (°C) | 387 |
| TBP 90% (°C) | 723 |

Les caractéristiques du catalyseur sont les suivantes :
- Catalyseur : zéolithe Y dispersée dans une matrice à base de silice alumine
- Diamètre moyen du catalyseur : 70 micromètres
- Masse volumique du grain : 1250 kg/m3

La pression dans la zone réactionnelle est égale à 0,2 MPa et les conditions opératoires pour les marches "maxi propylène" et "maxi essence" sont les suivantes :

| Mode | Maxi propylène | Maxi essence |
|---|---|---|
| Température sortie réacteur (°C) | 550 | 528 |
| Rapport C/O (massique) | 7,5 | 7,1 |

Les rendements en propylène, coupe C4 et en essence par rapport à la charge selon les marches sont les suivants:

| Rendement (% poids de charge fraîche) | Maxi propylène | Maxi essence |
|---|---|---|
| Propylène | 7,2 | 4,8 |
| Coupe C4 | 13,7 | 9,8 |
| Essence (C5-220°C) | 44,1 | 47,1 |

### Exemple 2 (selon l'invention illustrée par la figure 1)

On traite le même résidu atmosphérique que dans l'exemple 1, dans le procédé selon l'invention en mode "maxi propylène", dans les conditions opératoires de craquage du résidu atmosphérique de l'exemple 1 "maxi propylène" (C/O=7,5 et 550°C en sortie de réacteur).

La coupe C4 de l'effluent du craquage catalytique est séparée dans la zone de séparation S1 puis S2, puis introduite dans le réacteur d'oligomérisation R2 qui fonctionne dans les conditions suivantes :
- Pression : 6,0 MPa
- Température : 140-160°C
- Vitesse spatiale : 0,5 à 1 h⁻¹

Le catalyseur d'oligomérisation est de la silice alumine amorphe.

Environ 83% poids des oléfines C4 sont oligomérisées principalement en oléfines C8.

Les oligomères, séparés des oléfines n'ayant pas réagi et des paraffines en C4 dans la zone de séparation S3, sont totalement recyclés dans le réacteur R1 de craquage catalytique.

Le craquage de ces oligomères permet d'augmenter le rendement en propylène et les oligomères non craqués viennent accroître le rendement global en essence.

Les rendements en propylène, coupe C4 et essence dans la marche maxi propylène rapportés à la charge de résidu atmosphérique sont les suivants :

| Rendement (% poids de charge fraîche) | Invention selon Fig. 1 |
|---|---|
| Propylène | 9,3 |
| Coupe C4 | 5,9 |
| Essence (C5-220°C) | 49,0 |

On observe donc par rapport à la marche "maxi propylène" selon l'art antérieur une augmentation de 2 points (9,3 - 7,2) sur le propylène et simultanément de 5 points (49- 44,1) sur l'essence.

### Exemple 3 (selon l'invention illustrée par la figure 2)

On traite toujours le même résidu atmosphérique dans le procédé selon l'invention en mode "maxi essence", dans les conditions opératoires de craquage du résidu atmosphérique de l'exemple 1 "maxi essence" (C/O=7,1 et 528°C en sortie de réacteur).

La coupe C3 + C4, séparée dans la zone de séparation S1, est introduite dans le réacteur d'oligomérisation R2 qui fonctionne dans les conditions suivantes :
- Pression : 6,6 MPa
- Température : 130-160°C
- Vitesse spatiale : 0,5 -1 h⁻¹

Le catalyseur d'oligomérisation utilisé est également de la silice alumine amorphe.
Environ 87% poids des oléfines C3 + C4 sont oligomérisées en oléfines C8, C9 et C12.
Les oligomères, séparés des oléfines n'ayant pas réagi et des paraffines dans la zone de séparation S3, s'ajoutent à l'essence issue du craquage du résidu atmosphérique et une très faible part au pool gazole.

Les rendements en propylène, coupe C4 et essence selon l'invention en marche "maxi essence" '(illustrée par la figure 2), rapportés à la charge de résidu atmosphérique sont les suivants :

| Rendement (% poids de charge fraîche) | Invention selon Fig. 2 |
|---|---|
| Propylène | 0,2 |
| Coupe C4 | 4,2 |
| Essence (C5-220°C) | 55,2 |

Le rendement en essence est augmenté de 8 points (55,2- 47,1) par rapport à la marche maxi essence selon l'art antérieur.

## Revendications

1. Procédé de coproduction d'essence et de propylène à partir d'une charge hydrocarbonée lourde de point d'ébullition initial supérieur ou égal à 340°C faisant appel à une unité de craquage catalytique suivie d'une unité d'oligomérisation pouvant fonctionner selon deux marches dite "maxi propylène", et "maxi essence" dans lequel,
a) pour la marche "maxi propylène", la charge de l'unité d'oligomérisation est constituée de la coupe C4 ou de la coupe C4/C5 oléfinique issue du craquage catalytique, et les effluents de l'unité d'oligomérisation sont séparés en une coupe d'oligomères en C8+ qui est au moins en partie recyclée à l'entrée de l'unité de craquage catalytique (dite charge légère), la coupe essence du procédé étant constituée de la coupe essence produite à l'unité de craquage catalytique et de la coupe oligomères issue de l'unité d'oligomérisation qui n'est pas recyclée au craquage catalytique, le craquage catalytique étant effectué:
- soit dans un seul réacteur à écoulement ascendant, la température de sortie réacteur (ROT) étant comprise entre 510 et 580°C, préférentiellement comprise entre 540 et 590°C, et le rapport C/O est compris entre 8 et 20,
- soit dans deux réacteurs à écoulement ascendant, le premier réacteur FCC effectuant le craquage de la charge lourde travaillant à une température de sortie réacteur (ROT1) comprise entre 510°C et 580°C, de préférence comprise entre 530 et 560°C, et un rapport C/O compris entre 5 et 10, et le second réacteur FCC effectuant le craquage des oligomères en C8+ issus de l'unité d'oligomérisation (dite charge légère), travaillant à une température de sortie réacteur (ROT2) comprise entre 550°C et 650°C, préférentiellement comprise entre 570 et 620°C, avec un rapport C/O compris entre 8 et 25, et l'unité d'oligomérisation fonctionnant à une température comprise entre 100°C, et 200 °C, préférentiellement comprise entre 140°C et 160°C, et à une pression comprise entre 0,5 et 10 Mpa,
b) pour la marche "maxi essence", la charge de l'unité d'oligomérisation est constituée de la coupe C3/C4 oléfinique issue du craquage catalytique, et les effluents de l'unité d'oligomérisation sont séparés en une coupe d'oligomères en C6+ qui est ajoutée à la coupe essence issue du craquage catalytique pour constituer l'essence produite par le procédé, le propylène étant obtenu dans les deux marches à partir des effluents du FCC après séparation dans une ou plusieurs colonnes à distiller, le craquage catalytique étant effectué dans un ou deux réacteurs travaillant en écoulement ascendant, la température de sortie (ROT) étant comprise entre 500°C et 580°C, préférentiellement comprise entre 520°C et 550°C, et le rapport C/O est compris entre 5 et 10, le craquage catalytique étant effectué dans un ou deux réacteurs travaillant en écoulement ascendant, la température de sortie (ROT) étant comprise entre 500°C et 580°C, préférentiellement comprise entre 520°C et 550°C, et le rapport C/O est compris entre 5 et 10,
et l'unité d'oligomérisation fonctionnant à une température comprise entre 100°C et 200°C, préférentiellement comprise entre 140°C et 160°C, et à une pression comprise entre 2 et 10 MPa,
ledit procédé pouvant fonctionner selon les deux marches précédemment définies en basculant au cours du temps de l'une à l'autre, ainsi que selon toute marche intermédiaire.

2. Procédé de coproduction d'essence et de propylène à partir d'une charge hydrocarbonée lourde de point d'ébullition initial supérieur ou égal à 340°C faisant appel à une unité de craquage catalytique suivie d'une unité d'oligomérisation pouvant fonctionner selon deux marches dite "maxi propylène", et "maxi essence" dans lequel,
a) pour la marche "maxi propylène", la charge de l'unité d'oligomérisation est constituée de la coupe C4 ou de la coupe C4/C5 oléfinique issue du craquage catalytique, et les effluents de l'unité d'oligomérisation sont séparés en une coupe d'oligomères en C8+ qui est au moins en partie recyclée à l'entrée de l'unité de craquage catalytique (dite charge légère), la coupe essence du procédé étant constituée de la coupe essence produite à l'unité de craquage catalytique et de la coupe oligomères issue de l'unité d'oligomérisation qui n'est pas recyclée au craquage catalytique, le craquage catalytique étant effectué :
- soit dans un seul réacteur à écoulement descendant, la température de sortie réacteur (ROT) est comprise entre 550 et 700°C, et le rapport C/O est compris entre 15 et 50,
- soit dans deux réacteurs descendants, le premier réacteur FCC effectuant le craquage de lacharge lourde travaillant à une température de sortie réacteur (ROT1) comprise entre 550°c et 700°C, et un rapport C/O compris entre 15 et 50, et le second réacteur FCC effectuant le craquage des oligomères en C8+ issus de l'unité d'oligomérisation (dite charge légère), travaillant à une température de sortie réacteur (ROT2) comprise entre 570°C et 700°C, avec un rapport C/O compris entre 15 et 50, et l'unité d'oligomérisation fonctionnant à une température comprise entre 100°C et 200°C, préférentiellement comprise entre 140°C et 160°C, et une pression comprise entre 0,5 et 10 Mpa,
b) pour la marche "maxi essence", la charge de l'unité d'oligomérisation est constituée de la coupe C3/C4 oléfinique issue du craquage catalytique, et les effluents de l'unité d'oligomérisation sont séparés en une coupe d'oligomères en C6+ qui est ajoutée à la coupe essence issue du craquage catalytique pour constituer l'essence produite par le procédé, le propylène étant obtenu dans les deux marches à partir des effluents du FCC après séparation dans une ou plusieurs colonnes à distiller, le craquage catalytique étant effectué dans un ou deux réacteurs travaillant en écoulement descendant, la température de sortie (ROT) étant comprise entre 530°C et 650°C, et le rapport C/O est compris entre 15 et 25, et l'unité d'oligomérisation fonctionnant à une température comprise entre 100°C et 200°C, préférentiellement comprise entre 140°C et 160°C, et à une pression comprise entre 2 et 10 MPa,
ledit procédé pouvant fonctionner selon les deux marches précédemment définies en basculant au cours du temps de l'une à l'autre, ainsi que selon toute marche intermédiaire.

3. Procédé de coproduction d'essence et de propylène à partir d'une charge hydrocarbonée de point d'ébullition initial supérieur à 340°C selon l'une quelconque des revendications 1 à 2, dans lequel la coupe C4, ou la coupe C4/C5, ou la coupe C3/C4 est envoyée dans une unité d'hydrogénation sélective située en amont de l'unité d'oligomérisation, ladite unité d'hydrogénation sélective fonctionnant à une température comprise entre 0 et 200°C, une pression comprise entre 0,5 et 5 MPa, une vitesse spatiale comprise entre 0,5 et 5 m3 par heure et par m3 de catalyseur, et le rapport molaire H2/(composés acétyléniques + dioléfiniques) étant compris entre 1 et 3.

4. Procédé de coproduction d'essence et de propylène à partir d'une charge hydrocarbonée de point d'ébullition initial supérieur à 340°C selon l'une quelconque des revendications 1 à 2, dans lequel le catalyseur utilisé dans le réacteur de craquage catalytique est constitué d'une zéolithe de type Y ultra stable dispersée dans une matrice d'alumine, de silice, ou de silice alumine, à laquelle on ajoute un additif à base de zéolithe ZSM5, la quantité en cristaux de ZSM5 dans l'inventaire total étant inférieure à 10% poids.

## Patentansprüche

1. Verfahren zur gemeinsamen Produktion von Benzin und Propylen ausgehend von einer Charge aus schwerem Kohlenwasserstoff mit anfänglichem Siedepunkt größer oder gleich 340 °C unter Heranziehung einer katalytischen Crackingeinheit gefolgt von einer Oligomerisationseinheit, die gemäß zwei Betriebsarten, "Maxi Propylen" und "Maxi Benzin" genannt, funktionieren kann, wobei
a) für die Betriebsart "Maxi Propylen" die Charge der Oligomerisationseinheit aus der Fraktion C4 oder der olefinischen Fraktion C4/C5, die aus dem katalytischen Cracken hervorgeht, besteht, und die Abflüsse der Oligomerisationseinheit in eine Oligomerfraktion C8+ getrennt werden, die mindestens teilweise zu dem Eingang der katalytischen Crackingeinheit zurückgeführt wird (leichte Charge genannt), wobei die Benzinfraktion des Verfahrens aus der Benzinfraktion, die an der katalytischen Crackingeinheit erzeugt wird, und der Oligomerfraktion, die aus der Oligomerisationseinheit hervorgeht, die nicht zu dem katalytischen Cracking zurückgeführt wird, besteht, wobei das katalytische Cracken wie folgt durchgeführt wird:
- entweder in einem einzigen Reaktor mit aufsteigender Strömung, wobei die Reaktorausgangstemperatur (ROT) zwischen 510 und 580 °C liegt, bevorzugt zwischen 540 und 590 °C, und das C/O-Verhältnis zwischen 8 und 20 liegt,
- oder in zwei Reaktoren mit aufsteigender Strömung, wobei der erste Reaktor FCC das Cracken der schweren Charge ausführt, indem er mit einer Reaktorausgangstemperatur (ROT1) zwischen 510 °C und 580 °C, bevorzugt zwischen 530 und 560 °C und einem C/O-Verhältnis zwischen 5 und 10 arbeitet, und wobei der zweite Reaktor FCC das Cracken der Oligomere C8+, die aus der Oligomerisationseinheit hervorgehen (leichte Charge genannt) ausführt, indem er mit einer Reaktorausgangstemperatur (ROT2) zwischen 550 °C und 650 °C, bevorzugt zwischen 570 und 620 °C, mit einem C/O-Verhältnis zwischen 8 und 25 arbeitet,
und die Oligomerisationseinheit mit einer Temperatur zwischen 100 °C und 200 °C, bevorzugt zwischen 140 °C und 160 °C, und einem Druck zwischen 0,5 und 10 MPa arbeitet,
b) für die Betriebsart "Maxi Benzin" die Charge der Oligomerisationseinheit aus der olefinischen Fraktion C3/C4 besteht, die aus dem katalytischen Cracken hervorgeht, und die Abflüsse der Oligomerisationseinheit in eine Oligomerfraktion C6+ getrennt werden, die zu der Benzinfraktion, die aus dem katalytischen Cracken hervorgeht, hinzugefügt wird, um das von dem Verfahren erzeugte Benzin zu bilden,
wobei das Propylen bei den zwei Betriebsarten ausgehend von den Abflüssen des FCC nach dem Trennen in einer oder mehreren Destillationssäulen erhalten wird, wobei das katalytische Cracken in einem oder zwei Reaktoren ausgeführt wird, die mit aufsteigender Strömung arbeiten, wobei die Ausgangstemperatur (ROT) zwischen 500 °C und 580 °C liegt, bevorzugt zwischen 520 °C und 550 °C, und das C/O-Verhältnis zwischen 5 und 10 liegt, wobei das katalytische Cracken in einem oder zwei Reaktoren ausgeführt wird, die mit aufsteigender Strömung arbeiten, wobei die Ausgangstemperatur (ROT) zwischen 500 °C und 580 °C, bevorzugt zwischen 520 °C und 550 °C liegt, und das C/O-Verhältnis zwischen 5 und 10 liegt,
und wobei die Oligomerisationseinheit mit einer Temperatur zwischen 100 °C und 200 °C, bevorzugt zwischen 140 °C und 160 °C, und mit einem Druck zwischen 2 und 10 MPa arbeitet,
wobei das Verfahren gemäß den zwei oben definierten Betriebsarten unter Umwechseln im Laufe der Zeit von dem einen zu dem anderen sowie gemäß jedem Zwischenbetrieb funktionieren kann.

2. Verfahren zur gemeinsamen Produktion von Benzin und Propylen ausgehend von einer Charge aus schwerem Kohlenwasserstoff mit anfänglichem Siedepunkt größer oder gleich 340 °C unter Heranziehung einer katalytischen Crackingeinheit gefolgt von einer Oligomerisationseinheit, die gemäß zwei Betriebsarten, "Maxi Propylen" und "Maxi Benzin" genannt, funktionieren kann, wobei
a) für die Betriebsart "Maxi Propylen" die Charge der Oligomerisationseinheit aus der Fraktion C4 oder der olefinischen Fraktion C4/C5, die aus dem katalytischen Cracken hervorgeht, besteht, und die Abflüsse der Oligomerisationseinheit in eine Oligomerfraktion C8+ getrennt werden, die mindestens teilweise zu dem Eingang der katalytischen Crackingeinheit zurückgeführt wird (leichte Charge genannt), wobei die Benzinfraktion des Verfahrens aus der Benzinfraktion, die an der katalytischen Crackingeinheit erzeugt wird, und der Oligomerfraktion, die aus der Oligomerisationseinheit hervorgeht, die nicht zu dem katalytischen Cracking zurückgeführt wird, besteht, wobei das katalytische Cracken wie folgt durchgeführt wird:
- entweder in einem einzigen Reaktor mit absteigender Strömung, wobei die Reaktorausgangstemperatur (ROT) zwischen 550 und 700 °C liegt und das C/O-Verhältnis zwischen 15 und 50 liegt,
- oder in zwei Reaktoren mit absteigender Strömung, wobei der erste Reaktor FCC das Cracken der schweren Charge ausführt, indem er mit einer Reaktorausgangstemperatur (ROT1) zwischen 550 °C und 700 °C und einem C/O-Verhältnis zwischen 5 und 50 arbeitet, und wobei der zweite Reaktor FCC das Cracken der Oligomere C8+, die aus der Oligomerisationseinheit hervorgehen (leichte Charge genannt), ausführt, indem er mit einer Reaktorausgangstemperatur (ROT2) zwischen 570 °C und 700 °C mit einem C/O-Verhältnis zwischen 15 und 50 arbeitet,
und die Oligomerisationseinheit mit einer Temperatur zwischen 100 °C und 200 °C, bevorzugt zwischen 140 °C und 160 °C und mit einem Druck zwischen 0,5 und 10 MPa arbeitet,
b) für die Betriebsart "Maxi Benzin" die Charge der Oligomerisationseinheit aus der olefinischen Fraktion C3/C4 besteht, die aus dem katalytischen Cracken hervorgeht, und die Abflüsse der Oligomerisationseinheit in eine Oligomerfraktion C6+ getrennt werden, die zu der Benzinfraktion, die aus dem katalytischen Cracken hervorgeht, hinzugefügt wird, um das von dem Verfahren erzeugte Benzin zu bilden, wobei das Propylen bei den zwei Betriebsarten ausgehend von den Abflüssen des FCC nach dem Trennen in einer oder mehreren Destillationssäulen erhalten wird, wobei das katalytische Cracken in einem oder zwei Reaktoren ausgeführt wird, die mit absteigender Strömung arbeiten, wobei die Ausgangstemperatur (ROT) zwischen 530 °C und 600 °C liegt und das C/O-Verhältnis zwischen 15 und 25 liegt, wobei die Oligomerisationseinheit mit einer Temperatur zwischen 100 °C und 200 °C, bevorzugt zwischen 140 °C und 160 °C, und einem Druck zwischen 2 und 10 MPa arbeitet, wobei das Verfahren gemäß den zwei oben definierten Betriebsarten unter Umwechseln im Laufe der Zeit von dem einen zu dem anderen sowie gemäß jedem Zwischenbetrieb funktionieren kann.

3. Verfahren zur gemeinsamen Produktion von Benzin und Polypropylen ausgehend von einer Kohlenwasserstoffcharge mit anfänglichem Siedepunkt größer als 340 °C nach einem der Ansprüche 1 bis 2, wobei die Fraktion C4 oder die Fraktion C4/C5 oder die Fraktion C3/C4 in eine Einheit zur selektiven Hydrierung gesendet wird, die stromaufwärts der Oligomerisationseinheit liegt, wobei die Einheit zur selektiven Hydrierung mit einer Temperatur zwischen 0 und 200 °C, einem Druck zwischen 0,5 und 5 MPa, einer Raumgeschwindigkeit zwischen 0,5 und 5 m3 pro Stunde und pro m3 Katalysator funktioniert und das Molverhältnis H2/(Acetylen-Verbindungen + Diolefine) zwischen 1 und 3 liegt.

4. Verfahren zur gemeinsamen Produktion von Benzin und Polypropylen ausgehend von einer Charge aus Kohlenwasserstoff mit anfänglichem Siedepunkt größer als 340 °C nach einem der Ansprüche 1 bis 2, wobei der Katalysator, der in dem Reaktor zu dem katalytischen Cracken verwendet wird, aus einem Zeolith des ultrabeständigen Typs Y dispergiert in einer Tonerde-, Siliziumdioxid oder Silizium-Aluminiumoxid besteht, zu dem ein Zusatzstoff auf der Basis von Zeolith ZSM5 hinzugefügt wird, wobei die Menge an ZSM5-Kristallen in dem Gesamtbestand niedriger ist als 10 Gew.-%.

## Claims

1. A process for the co-production of gasoline and propylene from a heavy hydrocarbon feed with an initial boiling point of more than 340°C using a catalytic cracking unit followed by an oligomerization unit which can function in accordance with two regimes termed "maxi propylene" and "maxi gasoline", in which:
• for the "maxi propylene" regime, the feed for the oligomerization unit is constituted by the C4 cut or the C4/C5 olefinic cut derived from catalytic cracking and the effluents from the oligomerization unit are separated into a C8+ oligomer cut which is recycled at least in part to the inlet to the catalytic cracking unit, the gasoline cut from the process being constituted by the gasoline cut produced in the catalytic cracking unit and the oligomer cut derived from the oligomerization unit which is not recycled for catalytic cracking, the catalytic cracking being realized:
• -either in a reactor with an ascendant flow, the outlet riser temperature (ROT) being in the range 510°C to 580°C, preferably in the range 540°C to 590°C, and the C/O being in the range from 8 to 20,
• -either in two reactors with ascendant flow, the first FCC reactor working on the heavy feed and running at an outlet riser temperature (ROT1) in the range 510°C to 580°C, preferably in the range 530°C to 560°C and a ratio C/O in the range 5 to 10, and the second FCC reactor achieving the cracking of the oligomers with more than 8 carbon atoms (C8+) coming from the oligomerization unit (feed called "light feed") and running at an outlet riser temperature (ROT2) in the range 550°C to 650°C, preferably in the range 570°C to 620°C, with a C/O ratio in the range 8 to 25, and the oligomerization unit operating at a temperature in the range 100°C to 200°C, preferably in the range 140°C to 160°C at a pressure in the range 0,5 to 10 MPa,
• for the "maxi gasoline" regime, the feed for the oligomerization unit is constituted by the olefinic C3/C4 cut derived from catalytic cracking, and the effluents from the oligomerization unit are separated into a C6+ oligomer cut which is added to the gasoline cut derived from catalytic cracking to constitute the gasoline produced by the process;
the propylene being obtained in the two regimes from FCC effluents after separation in one or more distillation columns, the catalytic cracking being realized in one or two reactors with an ascendant flow, the outlet temperature (ROT) being in the range 500°C to 580°C, preferably in the range 520°C to 550°C and with a C/O ratio in the range 5 to 10, and the oligomerization unit operating at a temperature in the range 100°C to 200°C, preferably in the range 140°C to 160°C, at a pressure in the range 2 to 10 MPa, said process being able to run according to the two regimes defined above by switching from one to the other at any time, or according to any intermediate regime.

2. A process for the co-production of gasoline and propylene from a hydrocarbon feed with an initial boiling point of more than 340°C, using a catalytic cracking unit followed by an oligomerization unit able to run according to two regimes termed "maxi propylene" and "maxi gasoline", in which:
• a) for the "maxi propylene" regime, the feed for the oligomerization unit is constituted by the C4 cut or the C4/C5 olefinic cut derived from catalytic cracking, and the effluents from the oligomerization unit are separated into a C8+ oligomer cut which is recycled at least in part to the inlet to the catalytic cracking unit, the gasoline cut from the process being constituted by the gasoline cut produced in the catalytic cracking unit and the oligomer cut derived from the oligomerization unit which is not recycled for catalytic cracking, the catalytic cracking being realized:
• -either in a reactor with an ascendant flow, the outlet riser temperature (ROT) being in the range 550°C to 700°C, and the C/O being in the range from 15 to 50,
• -either in two reactors with ascendant flow, the first FCC reactor working on the heavy feed and running at an outlet riser temperature (ROT1) in the range 550°C to 700°C, and a ratio C/O in the range 15 to 50, and the second FCC reactor achieving the cracking of the oligomers with more than 8 carbon atoms (noted C8+) coming from the oligomerization unit (feed called "light feed") and running at an outlet riser temperature (ROT2) in the range 570°C to 700°C, with a C/O ratio in the range 15 to 50, and the oligomerization unit operating at a temperature in the range 100°C to 200°C, preferably in the range 140°C to 160°C at a pressure in the range 0,5 to 10 MPa,
• b) for the "maxi gasoline" regime, the feed for the oligomerization unit is constituted by the olefinic C3/C4 cut derived from catalytic cracking, and the effluents from the oligomerization unit are separated into a C6+ oligomer cut which is added to the gasoline cut derived from catalytic cracking to constitute the gasoline produced by the process;
the propylene being obtained in the two regimes from FCC effluents after separation in one or more distillation columns, the catalytic cracking being realized in one or two reactors with an ascendant flow, the outlet temperature (ROT) being in the range 530°C to 650°C, and with a C/O ratio in the range 15 to 25, and the oligomerization unit operating at a temperature in the range 100°C to 200°C, preferably in the range 140°C to 160°C, at a pressure in the range 2 to 10 MPa, said process being able to run according to the two regimes defined above by switching from one to the other at any time, or according to any intermediate regime.

3. A process for the co-production of gasoline and propylene from a hydrocarbon feed with an initial boiling point of more than 340°C according to any one of claims 1 to 2, in which the C4 cut or the C4/C5 cut or the C3/C4 cut is sent to a selective hydrogenation unit located upstream of the oligomerization unit, said selective hydrogenation unit operating at a temperature in the range 0°C to 200°C, a pressure in the range 0.5 to 5 MPa, a space velocity in the range 0.5 to 5 m³ per hour and per m³ of catalyst, with the H₂/(acetylenic + diolefinic compounds) molar ratio being in the range 1 to 3.

4. A process for the co-production of gasoline and propylene from a hydrocarbon feed with an initial boiling point of more than 340°C according to any one of claims 1 to 2, in which the catalyst used in the catalytic cracking reactor is constituted by an ultra stable Y type zeolite dispersed in an alumina, silica, or silica-alumina matrix, to which an additive based on ZSM-5 zeolite is added, the total quantity of ZSM-5 crystals being less than 10% by weight.
